(19) **Europäisches Patentamt**

**European Patent Office**

**Office européen des brevets**

(11) **EP 1 348 697 A1**

(12) **EUROPEAN PATENT APPLICATION**

(43) Date of publication:
 **01.10.2003 Bulletin 2003/40**

(51) Int Cl.⁷: **C07D 211/90**, A61K 31/4422, A61P 9/00

(21) Application number: **02252309.6**

(22) Date of filing: **28.03.2002**

(84) Designated Contracting States:
 **AT BE CH CY DE DK ES FI FR GB GR IE IT LI LU MC NL PT SE TR**
 Designated Extension States:
 **AL LT LV MK RO SI**

(71) Applicant: **COUNCIL OF SCIENTIFIC & INDUSTRIAL RESEARCH**
 **New Delhi 110001 (IN)**

(72) Inventors:
 • **Joshi, Rohini Ramesh**
  **Maharashtra (IN)**

 • **Joshi, Ramesh Anna**
  **Maharashtra (IN)**
 • **Thottappillil, Ravindranathan**
  **Maharashtra (IN)**

(74) Representative: **Manaton, Ross Timothy**
 **JY & GW Johnson,**
 **Kingsbourne House,**
 **229-231 High Holborn**
 **London WC1V 7DP (GB)**

(54) **Process for the preparation of S(-)-amlodipine-L(+)-hemitartrate**

(57) The present invention relates to a process for the preparation of [S(-) amlodipine-L(+)-hemi tartrate] from RS amlodipine base using L(+) tartaric acid in the presence of an organic solvent such as dimethyl sulfoxide.

EP 1 348 697 A1

## Description

**Field of the invention**

The present invention relates to a process for the preparation of [S(-) amlodipine-L(+)-hemi tartrate] from RS amiodipine base using L(+) tartaric acid in the presence of an organic solvent such as dimethyl sulfoxide.

**Background of the invention**

Amlodipine and its salts are long acting calcium channel blockers and are useful for the treatment of cardiovascular disorders. Racemic Amiodipine is currently being used as its besylate in the treatment of hypertension and angina. The preparation of racemic compound is described in European patent 0089167. Amiodipine is racemic compound and has chiral center at 4 position of the dihydropyridine ring.

It has also been reported that the R(+) isomer is a potent inhibitor of smooth muscle cell migration (WO 95/05822). The S(-) isomer is having calcium channel blocker activity while the R(+) isomers has little or no calcium channel blocking activity.

Prior art for the preparation of R and S enantiomers of amlodipine are a) resolution of amlodipine azide ester with optically active 2-methoxy-2-phenylethanol (J. Med. Client, 29, 1696, 1986. J.E. Arrowsmith, S.F. Campbell, P.E. Cross, J.K. Stabs, R.A., Burges and EP Appl. 0331315A) or b) resolution of Amlodipine base with optically active camphanic acid [J. Med. Chem., 35, 3341, 1992, S. Goldman., J. Stoltefuss and L. Born) or c) resolution of RS.-amlodipine base to R(+) and S(-) isomer with L or D tartaric acid respectively in organic solvent DMSO {Peter L., Spargo US Patent 6, 046,338; (2000), WO 95/25722 (1995)] which indicate the use of both tartaric acids is essential.

**The disadvantages:**

The main disadvantages of the prior art are:

1. The use of unnatural tartaric acid for the separation of S(-) amlodipine
2. The use of costlier camphanic acid or 2-methoxy-2-phenylethanol as a resolving agents.

**Objects of the invention**

The main object of the invention is to develop a technology for the preparation of S(-) amiodipine from racemic amlodipine using naturally occurring L-tataric acid.

**Summary of the invention**

Accordingly, the invention provides a new and efficient process for the preparation of [S(-) amlodipine-L(+) hemi tartrate] in good yield with high enantiomeric purity by reacting RS amlodipine base with L(+) tartaric acid in an organic solvent at a temperature ranging from 20-35°C for a period ranging from 16 to 24 hours, separating by filtration solid [R(+) amlodipine-L(+)-hemi tartrate], seeding the filtrate to obtain solid [S(-) amlodipin-L(+)-hemi tartrate], filtering and recrystallising the solid, basifying to obtain S(-) amlodipine.

In one embodiment of the present invention the organic solvent used for the reaction is dimethyl sulfoxide.

In another embodiment of the present invention 0.5 mole of L(+) tartaric acid is used for the reaction.

In another embodiment the solvent used for crystallization is selected from the group consisting of methanol, ethanol and butanol.

In another embodiment of the invention basification is done using metal hydroxides, carbonates or aq. Ammonia.

**Detailed description of the invention**

The unique feature of the invention is preferential crystallization of enantiomer salt with respect to quantity of DMSO and time. The process of resolution of RS amlodipine using L(+) tartaric acid is shown in the scheme below :

(R,S) Amlodipine    L(+)TA        R(+) Amlodipine hemi L(+) TA

$\xrightarrow{\text{DMSO}}$        mono DMSO solvate

↳ → Filtrate

│ Seeding

↓

S(-) Amlodipine hemi L(+) TA mono DMSO solvate

│ aq. Ammonia/CH$_2$Cl$_2$

Benzene

S(-) Amlodipine besylate ◄ —— sulfonic acid —— S(-) Amlodipine

SCHEME

The process of the present invention is described herein below with reference to examples which are illustrative only and should not be construed to limit the scope of the present invention in any manner.

**EXAMPLE -1**

**Amlodipine hemi L tartarate-mono-DMSO solvate mp 160-162°C $[\alpha]^t$ = +24.32 (c=1, R(+) Amlodipine-hemi-L-tartarate mono DMSO solvate and S(-) Amlodipine-hemi-L tartarate mono DMSO solvate from (RS) Amlodipine.**

To a stirred solution of 10.50 gm (0.0256 mole), of RS Amiodipine in 30 ml of DMSO was added a solution of 1.93 (0.128) mole (0.5 equiv) of L(+) Tartaric acid in 30 ml DMSO. The solid starts separating from clear solution within 5-10 min. This was stirred for 3 hrs. and the solid was filtered off, washed with acetone and dried to give 6.66 gm, 46.15% R(+) MeOH). The filtrate was seeded with S(-) amiodipine hemi L(+) tartarate salt. and left overnight the solid was filtered off and washed with 10ml acetone and dried to give 6.41 gm, 44.4% S(-) amlodipine-hemi L(+)-tartarate mono DMSO solvate.mp 169.5-171.5°C = -14.1 (c=1, MeOH) 90% de by chiral HPLC. (J.Chrom., B 693, 367 (1997) J. Luksa, Dj. Josic, B. Podobinc, B. Furlan, M. Kremser]

**EXAMPLE -2**

**RS Amlodipine L(+) tartarate mono DMSO solvate from RS Amlodipine**

The procedure as described in example 1 was repeated and the reaction was kept overnight. The solid filtered and dried to yeidl 14 gm, 97.9% RS Amlodipien L(+) tartarate mono DMSO solvate. Mp 148.5-151°C (c=1 MeOH) 3.3% de by chiral HPLC.

**EXAMPLE-3**

**S(-) Amlodipine hemi L(+) tartarate monohydrate from S(-) Amlodipine-hemi-L-(+) tartarate monohydrate DMSO solvate - methanol as solvent.**

50 gms of S(-) Amlodipine-hemi-L(+)-tartarate mohohydrate DMSO solvate was dissolved in 250 ml refluxing methanol (30 min). The solution was kept overnight at room temperature (25-28°C) with stirring. The solid was collected by filtration, washed with 100 ml methanol and dried at 50°C in vacuo (2 hrs till constant wt.) to give 35 gm (80%). S (-) Amlodipine-hemi-L(+)-tartarate monohydrate. Mp 171-172°C = 114.1 (c=1, MeOH); 90% de chiral HPLC.

**EXAMPLE -4**

**S(-) Amlodipine hemi L(+)-tartarte mohohydrate from S(-) Amlodipine-hemi-L-(+) tartarate monohydrate DMSO solvate - Ethanol as solvent.**

The procedure was followed as mentioned in example 3 was using ethanol (150 ml) instead of methanol. The solid obtained was collected by filtration, washed with 50 ml cold ethanol and dried at 50°C in vacuo (2 hrs till constant wt.) to give 30 gms (68%). S(-) Amlodipine hemi L(+) tartarate monohydrate mp 172.5-174°C = 17.44 (C=1, MeOH), 97% de chiral HPLC.

**EXAMPLE -5**

**S(-) Amlodipine from (S) (-)amlodipine hemi L (+) tartarte mohonydrate.**

S(-) Amlodipine hemi L(+) tartarate mohohydrate (30 gms) was slurried in 60 ml $CH_2Cl_2$ and 60 ml (6%) aqueous ammonia for 30 min. The organic solution was separated and washed with water. The organic extract was dried to give solid. The solid was filtered and dried at room temperature under vacuo to give 20 gms (82%) S(-) amiodipine mp 108-109°C 30.55 (c=1, MeOH), 97.4% ee by chiral HPLC.

**EXAMPLE -6**

**S(-) Amlodipine from S(-) amlodipine hemi L(+) tartarte mono DMSO solvate**

S(-) Amlodipine hemi L(+) -tartarate mono DMSO solvate (30 gms) was slurried in 60 ml $CH_2Cl_2$ and 60 ml (6%) aqueous ammonia for 30 min. The organic solution was separated and washed with water. The organic extract was dried over anhydrous sodium sulphate and concentrated. The residue was triturated with hexane to give solid 20.1 gms (92%) S(-) amlodipine. Mp107-107.5°C 27.3 (c=1, MeOH), 90% ee by chiral HPLC.

**Claims**

1.  A process for the preparation of [S(-) amiodipine -L(+)-hemi tartrate] which comprises reacting RS amlodipine base with L(+) tartaric acid in an organic solvent separating the solid [R(+) amlodipin-L(+)- hemi tartrate] by filtration, seeding the filtrate to obtain solid [S(-) amlodipin-L(+)-hemi tartrate] by precipitation, filtering the solid and basifying to obtain [S(-) amlodipine -L(+)-hemi tartrate].

2.  A process as claimed in claim 1, wherein the RS amlodipine base is reacted with the L(+) tartaric acid for a period in the range 16 to 24 hours.

3.  A process as claimed in claim 1 or claim 2, wherein the RS amlodipine base is reacted with the L(+) tartaric acid at a temperature in the range 20-35°C.

4.  A process as claimed in any of claims 1 to 3, wherein the solvent is DMSO.

5.  A process as claimed in any preceding claim, wherein the solvent to amlodipine ratio is 5-6 ml/gm of amlodipine.

6.  A process as claimed in any preceding claim, wherein L-tartaric acid employed is about 0.5 mole per mole of amlodipine.

7.  A process as claimed in any preceding claim, wherein the solvate precipitated is S(-) amlodipine hemi L(+)-tartrate mono DMSO solvate.

8.  A process claimed in claim 1 wherein a stirred solution of RS Amlodipine in DMSO is added to a solution of L(+) Tartaric acid in DMSO, the solid obtained separated by filtration, washed with acetone, dried to give R(+) MeOH), the filtrate seeded with S(-) amlodipine hemi L(+) tartarate salt, the solid so obtained filtered off and washed with acetone and dried to give S(-) amiodipine-hemi L(+)-tartarate mono DMSO solvate.

# EP 1 348 697 A1

**European Patent Office**

## EUROPEAN SEARCH REPORT

Application Number

EP 02 25 2309

| DOCUMENTS CONSIDERED TO BE RELEVANT | | | | |
|---|---|---|---|---|
| Category | Citation of document with indication, where appropriate, of relevant passages | | Relevant to claim | CLASSIFICATION OF THE APPLICATION (Int.Cl.7) |
| X | EP 1 181 932 A (PFIZER LTD ;PFIZER (US)) 27 February 2002 (2002-02-27) example 1(1), 2 --- | | 1-8 | C07D211/90 A61K31/4422 A61P9/00 |
| A,D | WO 95 25722 A (PFIZER LTD ;PFIZER RES & DEV (IE); PFIZER (US); SPARGO PETER LIONE) 28 September 1995 (1995-09-28) * page 7; example 5 * ----- | | 1 | |
| | | | | **TECHNICAL FIELDS SEARCHED    (Int.Cl.7)** C07D A61K A61P |

The present search report has been drawn up for all claims

| Place of search | Date of completion of the search | Examiner |
|---|---|---|
| THE HAGUE | 28 August 2002 | Bosma, P |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

6

**ANNEX TO THE EUROPEAN SEARCH REPORT
ON EUROPEAN PATENT APPLICATION NO.**

EP 02 25 2309

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2002

| Patent document cited in search report | | | Publication date | Patent family member(s) | | Publication date |
|---|---|---|---|---|---|---|
| EP 1181932 | A | | 27-02-2002 | BR 0103434 A | | 26-03-2002 |
| | | | | EP 1181932 A2 | | 27-02-2002 |
| | | | | JP 2002114683 A | | 16-04-2002 |
| | | | | US 2002045648 A1 | | 18-04-2002 |
| WO 9525722 | A | | 28-09-1995 | AT 166050 T | | 15-05-1998 |
| | | | | AU 677765 B2 | | 01-05-1997 |
| | | | | AU 1949395 A | | 09-10-1995 |
| | | | | BR 9507137 A | | 30-09-1997 |
| | | | | CA 2186263 A1 | | 28-09-1995 |
| | | | | CN 1144523 A ,B | | 05-03-1997 |
| | | | | CZ 9602784 A3 | | 12-03-1997 |
| | | | | DE 69502486 D1 | | 18-06-1998 |
| | | | | DE 69502486 T2 | | 10-09-1998 |
| | | | | DK 751938 T3 | | 07-10-1998 |
| | | | | WO 9525722 A1 | | 28-09-1995 |
| | | | | EP 0751938 A1 | | 08-01-1997 |
| | | | | ES 2116737 T3 | | 16-07-1998 |
| | | | | FI 963775 A | | 23-09-1996 |
| | | | | HU 76290 A2 | | 28-07-1997 |
| | | | | IL 113008 A | | 14-07-1999 |
| | | | | JP 2843681 B2 | | 06-01-1999 |
| | | | | JP 9510707 T | | 28-10-1997 |
| | | | | KR 188980 B1 | | 01-06-1999 |
| | | | | NO 963991 A | | 19-11-1996 |
| | | | | NZ 282404 A | | 24-11-1997 |
| | | | | PL 316535 A1 | | 20-01-1997 |
| | | | | RU 2132845 C1 | | 10-07-1999 |
| | | | | TW 448156 B | | 01-08-2001 |
| | | | | US 6046338 A | | 04-04-2000 |
| | | | | US 5750707 A | | 12-05-1998 |
| | | | | ZA 9502362 A | | 23-09-1996 |